# EUROPEAN PATENT APPLICATION

(11) **EP 1 043 002 A2**
(43) Date of publication of application: **11.10.2000**
(21) Application number: 99600022.0
(22) Date of filing: 28.12.1999
(51) Int. Cl.: A61F 2/44

(54) **Intervertebral disc replacement prosthesis**

(30) Priority: 07.04.1999 GR 99100116
(71) Applicant: Kalaitzis, Christos, 54642 Thessaloniki (GR); Karavelis, Antonios, 55131 Thessaloniki (GR)
(72) Inventor: Kalaitzis, Christos, 54642 Thessaloniki (GR); Karavelis, Antonios, 55131 Thessaloniki (GR)

(57) **Abstract**

Intervertebral disc replacement prosthesis consisting of a cylindrical cage and two securing pitch forks that can be placed in the inter-vertebral space to desired heights wherever in the Human Spine with anterior approach and, also on the Lumbar Spine with posterior approach in order to increase the total contact surface of the prosthesis. Only two instruments are demanded for its implantation and minimum time, it allows easy and constant optical checking of the whole procedure, due to less bleeding on the operating field and also due to the fact that no bulky instruments, guides etc. are used, and it finally combines the characteristics of both types of cages, cylindrical and flat ones, while it abolishes the disadvantages that each type has thus forcing surgeons to resort in positioning posterior fixation with pedicular screws at the same time for more safety.

## Description

### Terms used.

Arthrodesis of human spine, end plate, pitch fork

### B) TECHNICAL FIELD

Intervertebral prosthesis consisting of screwing cylindrical and conical cage, which after the screwing in the space between two vertebrae of the human spine, allows the connection on both its sides with two pitch forks, thus creating an assembly prosthesis, which is placed in the space between two vertebrae , in the middle line which separates the vertebral body into two equal parts, by anterior or posterior approach, that scopes to 1) successfully restore the inter-vertebral space's height, 2) effective and immediate immobilization and 3) arthrodesis and creation of the best conditions of permanent osteosynthesis consisting of the bone bridge connecting the two vertebral bodies and the implant which after the insertion is filled with autologous or heterologous graft.

### C) EVALUATION AND DESCRIPTION OF THE PREVIOUS TECHNIQUE

The need for arthrodesis of the human spine is widely mentioned in International bibliography and mostly in case of instability, degenerated inter-vertebral disc, spondylolishesis, etc. In order to succeed in it, we either insert autologous or heterologous graft, or even without posterior osteosynthesis with transpedicular screws and it is during the last decade that screwing hollow cages mostly made of titanium have been widely used while solving many of the previous techniques' problems and, thus, being appreciated by surgeons. However, their insertion technique because of their design has brought up some very serious problems whose correction is not always easy to accomplish. The most serious problem is that very precise handling of a series of sharp and dangerous instruments who are used only a few millimeters away or even in contact to tissues vital for life itself or for the patient's safety such as the aorta, the nerves' roots, the esophagus and others, without an easy control of those instruments' position and direction throughout the operation. It is due to this fact, that a plethora of complications occurs, such as vital tissue injuries, implantation in wrong position, or loose implantation without the desired opposite stabilization forces between the vertebral bodies. It is the creation of such forces that has to be pursued during implants' insertion, both to ensure stable placement and arthrodesis . In general with the existing technology according the International literature there is approximately a 20 % complication' s rate. "Reported disadvantages of threaded cages include luck of stability without pedicle screws, subsidence, lose of bone stock, difficulty in achieving lordosis etc. Conlusion : smaller cages with pedicle screws are more effective" see attached sheets NASS 99-Chicago.

The technical procedure of inserting implants of similar type like Ray, BAK-L etc., requires 'ground's (bone bed) preparation of the vertebral bodies' end plates by using very sharp instruments such as reamers, taps etc. that pass through tubular guides that usually carry teeth that are secured on the upper and lower body by lightly striking them, having previously distracted the gap's height with metal distractors of cylindrical or conical shape which are removed after fixation of the tubular teethed guide, thus, keeping the desired height after the removal of the distractor, but only through the guide's teeth. Then, though this tubular guide, we pass the reamer, the tap, and finally screw the cylindrical or conical-cylindrical shaped implant. Problems often arise during instrument handling when much attention has to be paid in continuous checking and controlling of the guide's direction(usually by C-arm) since a slight change, which is very easy to happen due to the needed force applied mostly during reaming of the end plates that leads to movement of 2-3 teeth holding the guide in position which results in a not equal cutting of upper and lower end plate and creating two not parallel holes(implant insertion) that have to notably be parallel to the sagital and vertical axis of the disc, following the parallelity of the end plates of the vertebral bodies. There are other types of tubular guides - distractors (paddle tube type) which could be right oriented and follow easy the parallel direction of the end plates, but they have the same technique of using reamers, taps etc.. This drawback appears due to the fact of insufficient control of the guide while we employ many tools whose handling is a prerequisite of the safe implantation, but all the procedure is very complicated and seriously increases risks that can lead to injuries or failures.

Because of the difficulties described above, we may loose the right direction and not correctly preparing the insertion hole of the implant, besides some times other complications, as bleeding or arrhythmia etc., oblige the surgeon to shorten the operation time, thus, not allowing the time margins for radioscopic checking and corrections to be made.

Thus, one of the operation's risks is the need of revision the whole procedure in order to correct the implant's insertion hole, which is mandatory to be attempted by choice of the next implant of larger size, after having used the next larger reamer, tap, etc. but this is not always possible. The most usual cases are that the implant isn't fairly screwed on the upper and lower body, or that it is screwed only on one and simply touches the other which can affect the implantation's stability that is our primary goal in order to stop the pain, possibly release the nerve's root and of course it is immobilization that speeds up the final fusion and arthrodesis. An other disadvantage of the previous technique is that using sharp reamers we normally destroy the end plate's structure and endurance along all the length of the created hole and things are getting worse if you have to operate two adjacent levels, because this could jeopardize the endurance of the remaining spongiosa bone tissue between the two operated discs.

There are also rectangular and not screwing cages which are inserted between two vertebrae by wedging in by striking and several surgeons turned to this version, just in order to avoid the above mentioned complications and difficulties because they are easy put in, without the risks which characterize the screwing cage's implantation and its postoperative complications. However these cages have a high risk to migrate, so it is mandatory to secure them with additional posterior fixation devices as pedicular screws.

### D) ADVANTAGES OF OUR INVENTION

Our prosthesis consists of two parts, the cylindrical which is advanced by screwing and the rectangular pitch forks which are secured and connected by light striking with it. This combination manages to appear the positive properties of both types, as is very stable and not easy to migrate, while leaves intact the end plate of the vertebrae, because we don' t use reamers, that means it does not jeopardize the endurance of them and the entire structure of the vertebral body, thus decreasing the risk of collapsing which seems to be a major disadvantage of the screwing cages, as there are many references of penetration in the vertebrae, or breakage of them. The goal to obtain the appearance of these properties, less collapse - no migration, is possible to minimize considerably the need for posterior fixation with pedicular screws, that means less operation time, less risk, cost effective and in general less intra and post operative complications, because the posterior fixation was necessary to protect the screwing cages to collapse and the rectangular to migrate.
There is also difference based on simplification through reducing the number of steps required, no reamer is used neither tap while the distractor which is firmly kept in position serves as guide as well. The risk of incorrect placement of the implant is seriously reduced since it carries self-cutting threads enabling it to be screwed passing over the distractor-guide, on the direction that it has been given. The implant is of cylindrical shape and its front part carries saw-like teeth on its perimeter with which it advances further, as then the self cutting external threads starts rotating, which helps the implant to be pushed forward up to the spot of its final position that can differ according to the way we operate (anterior or posterior). The implant itself is employed as a tool capable to prepare the insertion site and at the same time it is propelled, it is screwed while cutting the bone, destroying at least 50% less the end plate, unlike the other screwing cages.

After removal of partial or of all the inter-vertebral disk and the exposure of the end plates at the implantation site, two tools are only used for the final implantation. The distractor-guide and the holder-screw.

This is the basic advantage of the new method that requires less steps to be taken accompanied by a reduction in the time needed by the previous technique. Constant checking of the many instruments used by the old technique and pass or even get in contact with vital tissues as those of the aorta, the nerves' roots, and other smaller vessels, etc. is not required. Thus, the surgeon can succeed in quick and sure handling, without mistakes as far as direction is concerned thus increasing the possibilities of a good outcome, without the statistically high probability of injury that is present in such operations.

By adding of the pitch forks 30 and because of its design which increases the total contact surface, we obtain to achieve the combination of stable implantation, a property of the screwing cages which do not migrate easily and the property of the rectangular cages which increase the contact surface and do not allow the collapse of the implant.
Another problem of the older method was preparing the bone bed where spirals were made then with the use of tap. The preparation depth was often smaller than the implant's length preventing it from going deeper and the implant was improperly screwed or it even destroyed the threads due to the surgeon's effort to screw it further than the prepared ground, leading to easy pull out and implantation failure, which then required revision of the procedure and effort to place implant of larger size.

Conclusively the advantages are the speed, the safety, the stable implantation and the considerable reduced risk of collapse and migration.

### e) REVELATION

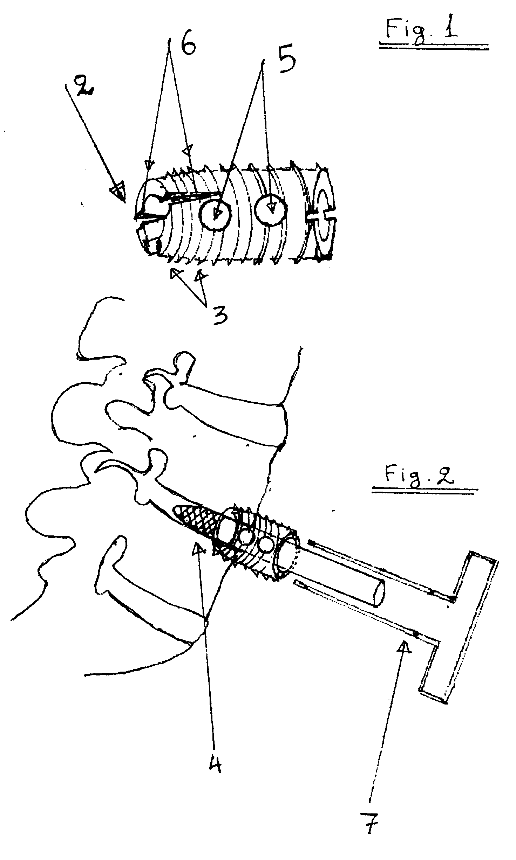

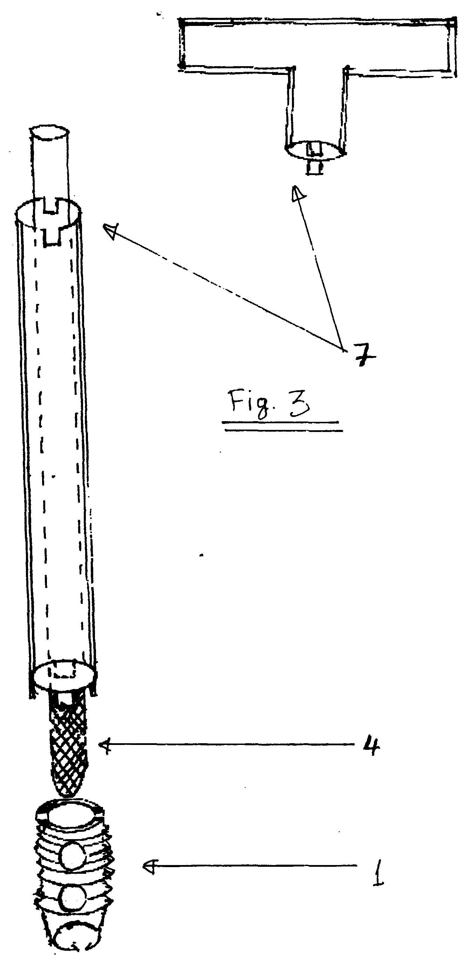

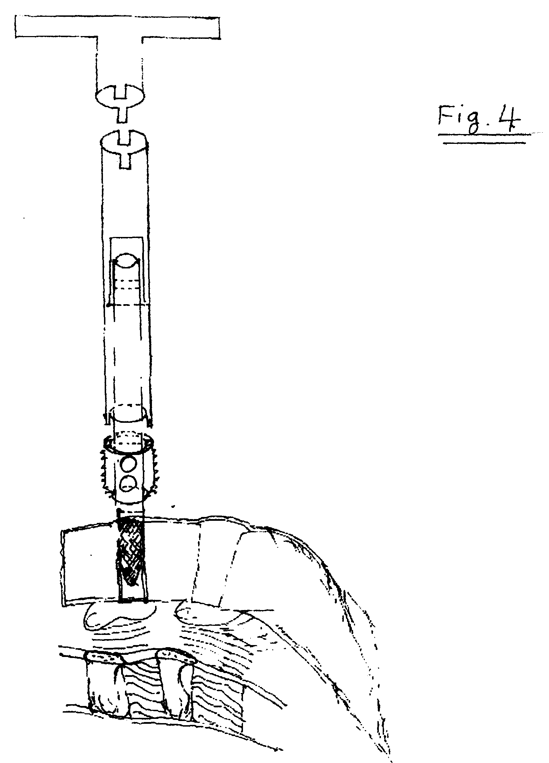

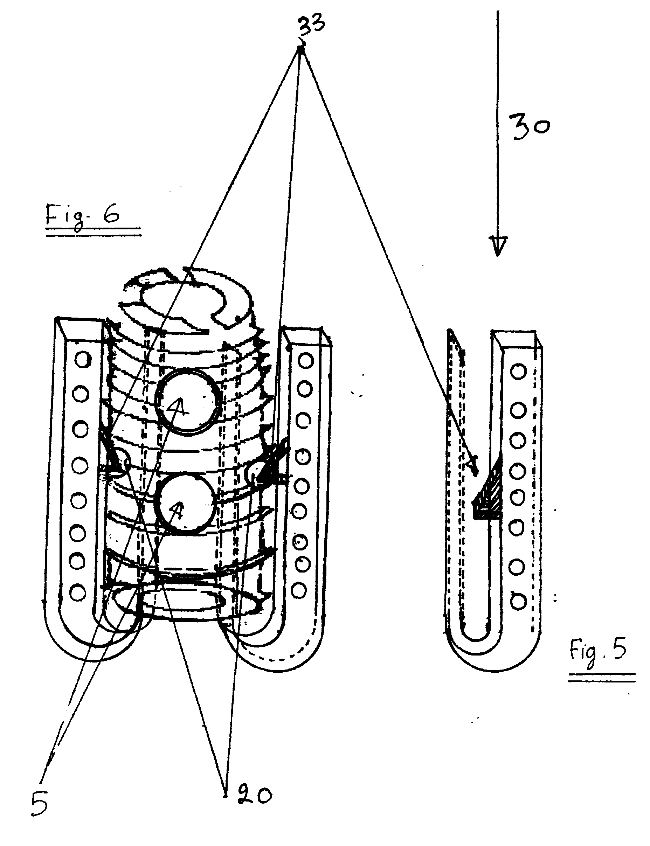

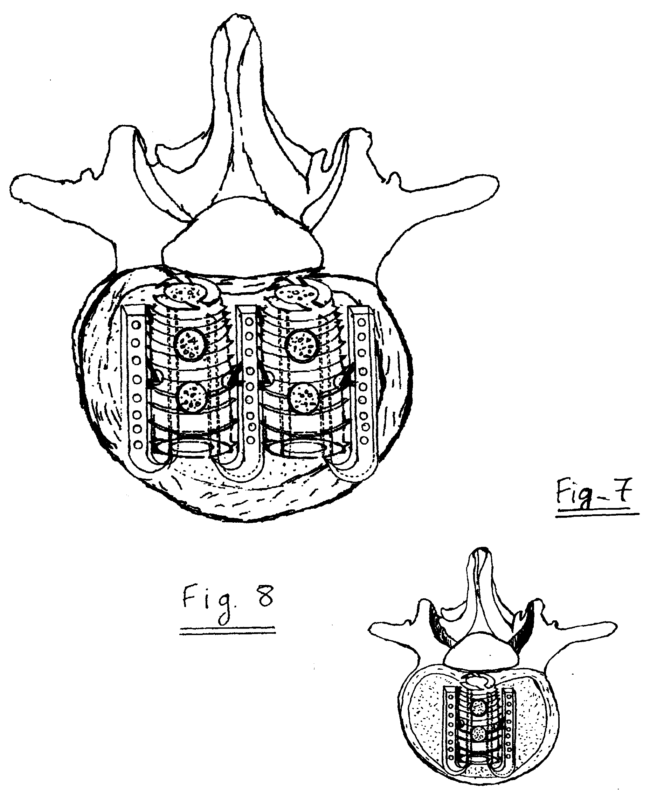

Fig. 1 shows a side view of the cylinder-implant, fig. 2 shows a side view of the vertebral gap where the implant is screwed in the opening where the damaged disk used to be. Fig. 3 shows the implant's divisible T holder and the distractor that passes through the T holder and through the cylinder-implant. Fig. 4 shows the steps of using the instruments. Fig. 5 shows the securing pitch forks, fig. 6 the assembled prosthesis, fig. 7 a transverse section of a single prosthesis implanted in the Intervertebral space, and fig. 8 a double one.
The inter-vertebral disc space prosthesis, consists of a round cylinder with external threads constructed of titanium alloy special for implants or of Carbon fibers, and of other material as well that are biocompatible and of certain hardness in order to withstand the loads and the forces, and a special pitch fork of the same material 30. Cylinder 1 bears saw-like teeth (2) on its front part that cut through the bone where it is implanted by application of screwing and relative pressure. Self-cutting threads 3 are an immediate continuity of the external teeth that allow its easy implantation through turning and under light pressure. Hence, the cylinder with its saw-like front part and the threads that follow immediately after it succeeds in being easily implanted since it cuts and is simultaneously screwed into the bone. The cylinder's internal section is round and of various diameters, that depend on the distractor that will be used and also depends on the level of the segment. For the Cervical spine distractors of smaller diameter are used, and one implant, while for Lumbar and Thoracic spine, 8-14mm and usually a double implant.
The cylinders have a diameter of 1-2mm bigger (thickness) than the diameter of the distractors which depends on the dimensions (size) of the implant-cylinder. All cylinders' sizes carry all around slots-openings 5 and two horizontal 20 fig. 6 which also are the pitch fork's securing positions.

The securing pitch fork 30, has a thin leg that passes through the internal part of the cylinder and another leg of such height and width so that it fills the gap between the two end plates of the upper and lower vertebral body which starts from 2mm for the Cervical spine and gets bigger for the Thoracic-Lumbar. It also carries teeth 33 in its internal section, which secures the cylinder and crotch's connection on the slot 20 of the cylinder.
The slots-openings on the cylinder and the crotch allow secondary development of bone tissue and the expectation of bridging the upper and lower vertebral bodies with bone bridge. The cylinder also carries fissures 6 on its front section into which the cut bone when screwing is accumulated thus not obstructing further screwing of the cylinder.

The only preparation demanded is positioning the distractor-guide 4, on an opening that has been created on the inter-vertebral disc with a drill 6-8mm, or removal of the disc with sharp curettes or disc forceps, until the end plates of the upper and lower body are revealed. Usage of distractors is common in all pre-existing techniques, due to the necessity of creating opposite stabilization forces that they create with their insertion. The advantages of our technique commence from this common point and then on since the distractor remains in position until the round cylinder which passes over it is screwed, and only then it is removed. Our cage is now securely screwed in the inter-vertebral space and the next step is to add and secure the pitch fork of necessary height which increases the whole contact area where the loads are exercised and generally we have the existence of the advantages of both types of cages, that is of cylindrical screwing ones and flat ones as well.
During positioning distractor 4 is not removed but remains in its position serves as a guide until the cylinder that passes over it is screwed. After the cylinder is screwed the distractor is removed.
Cylinder 1 passes over guide 4 is connected with the T holder 7 and is easily screwed by easily following guide's 4 route.

### EXAMPLE OF THE INVENTION'S APPLICATION

On a patient who has been suffering of back-pain for over than 6 months and alter clinical and laboratory examination with the indication of surgery in order to remove pain and restore its functionality, posterior exposure of the spine, laminectomy and total or partial cutting of the facet joints is performed, this is depends on the size of the implant that will be used by calculating the space between the cylinders on to which the double of their diameter is added.

With proportionate guide and below the dura we remove the disc's material at a 2,5 cm depth. We remove its remnants in order to reach the final upper and lower end plate of the corresponding vertebrae. We repeat the same procedure on the other side of the dura as well. Then we forward the distractor by hitting into position which will reduce the inter-vertebral space back to its normal while cautiously protecting the nerve's root and pushing aside the dura. We then check the space and whether there is space on the side for the proportionate implant without one touching the other. We pass a tubular holder of the implant over the distractor and we start to screw by applying slight pressure, through this procedure we forward the first of the two implants, then we repeat the same with the second one.
After the successful and secure implantation of the implant the distractor is removed from the interior of the tube-holder, the holder is also removed and the implant remains in position, before filling it with autograft or allograft, we position a pitch fork in between the two cages and one on each of the external sides, that is a total of three for the Lumbar spine. In case the cylinders are close and there is no space for the pitch fork between them only two are used, on their external sides. In the Cervical spine where usually one cage is employed, we have two pitch forks used one on its left and one on its right. In order to accomplish better stabilization we try to make the positioning as centrally as possible. In Thoracic spine it is possible to use a single implant that is longer and is placed diagonally due to the fact that the better approach is the transverse one, intrathoracic, or retro and the vertebral bodies are smaller.

We generally choose the pitch fork's height so that it attaches on the cage's side without affecting its stabilized implantation. This can occur if we distract during the pitch fork's implantation more than the distraction achieved by the screwing cage, that is why we try to use the most appropriate height. After this we can fill the cage's gap with graft.

In Lumbar spine implantation with transverse, or anterolateral approach we take care in identifying the middle line and on both of its sides we follow the same procedure with the only difference being that we have room especially in the space O5-I1 that allows us to have both distractors in position helping us to better estimate and control correct positioning regarding their in-between space.

## Claims

1. Intravertebral disc space prosthesis consisting of a hollow cylindrical cage, characterized of the conical front part and the external self cutting threads along its whole length, whose dimensions and sizes depends on the segment level of the spine we fuse, thus being longer and of larger diameter for the Lumbar spine and smaller for the corresponded Thoracic and Cervical, of about 1mm thickness, which cage has 3-4 fissures, which start from the front and narrow part of it where they shape sharp ends 2, Fig. 1, whose the internal hole is of same diameter at all of its length and smaller than the external one of about 2 mm, through of it passes the guide-distractor 4, whose cage the conical shape and sharp front ends allow it to be screwed over the distractor-guide 4, without any former bone bed preparation by using reamers and taps and after its implantation, allows the addition of the pitch forks 30, whose safety pin 33, secures it at the holes 5, at both of its sides, thus increasing the endurance of the system at loading forces and considerably decreasing the risk of the collapse and penetration of the prosthesis within the vertebral bodies.

2. Intravertebral disc space prosthesis as of claim 1, whose cylindrical cage front part's fissure's ends shape teeth like saws.

3. Intravertebral disc space prosthesis as of claim 1, whose external threads start immediately after the teeth like saws front sharp ends, thus shaping a whole threaded screw.

4. Intravertebral disc space prosthesis as of claim 1, of various sizes, whose internal hole where distractor-guide 4 passes, increases by 1 mm scale for any proportional external diameter.

5. Intravertebral disc space prosthesis as of claim 1, whose external diameter increases by 2 mm for any corresponded increase of its internal diameter hole and escalating size by 1mm of the distractor-guide 4 used.

6. Intravertebral disc space prosthesis as of claim 1, whose cylindrical cage has at its back part, two slots, where we apply the T-shape holder 7, Fig 2.

7. Intravertebral disc space prosthesis as of claim 1, which has the openings 5 at the upper and lower as well lateral sides, which allow the communication with the adjacent vertebral bodies, in order to facilitate the creation of bone bridge.

8. Intravertebral disc space prosthesis as of claim 1, whose securing pitch forks 30, has openings at its whole length, to allow and facilitate the creation of bone bridge.

9. Intravertebral disc space prosthesis as of claim 1, whose the securing pitch fork 30 has various sizes at whole of its dimensions, height, length and width, in order to fit at the right size of cylindrical screwing cage, thus fitting well for application at the Cervical, Thoracic and Lumbar spine.
